# EUROPEAN PATENT APPLICATION

(11) **EP 4 715 825 A1**
(43) Date of publication of application: **25.03.2026**
(21) Application number: 25202540.8
(22) Date of filing: 16.09.2025
(51) Int. Cl.: G16B 15/30, G16B 40/20, G16C 20/50, G16C 20/70

(54) **METHODS AND SYSTEMS FOR DYNAMIC DRUG DESIGN OF A PHARMACOLOGICAL TARGET**

(30) Priority: 18.09.2024 IN 202421070390
(71) Applicant: Tata Consultancy Services Limited, Mumbai, Maharashtra 400 021 (IN)
(72) Inventor: KALVA, Sukesh, 560096 Bangalore, Karnataka (IN); KARIM, S Abdul, 110001 New Delhi, Delhi (IN); PURANIK, Sarang Subhash, 560066 Bangalore, Karnataka (IN); SAXENA, Amit, 122004 Gurgaon, Haryana (IN)
(74) Representative: Goddar, Heinz J.

(57) **Abstract**

The disclosure relates generally to methods and systems for dynamic drug design of a pharmacological target. Conventional techniques in the drug design that use in-silico, in-vitro, and in-vivo approaches are not explicitly mentioned workflow related details for identifying novel molecules. The methods and systems of the present disclosure make the drug design dynamically by integrating the in-silico, in-vitro and in-vivo approaches through the dynamic generative artificial intelligence (GenAI) and artificial intelligence (AI) technologies. The integration of in-silico (molecular modeling and AI), in-vitro and in-vivo approaches helps in designing the novel optimized lead molecules. Optimization and prediction of ADMET based on QM based descriptors help in filtering the molecules.

## Description

### CROSS-REFERENCE TO RELATED APPLICATIONS AND PRIORITY

The present application claims priority from Indian application no. 202421070390, filed on September 18, 2024.

### TECHNICAL FIELD

The disclosure herein generally relates to the field of drug design, and more specifically to methods and systems for dynamic drug design of a pharmacological target.

### BACKGROUND

Drug design involves creating new medications based on the knowledge of a pharmacological or a biological target. Conventional techniques for designing a novel molecule with essential properties at an early stage of the drug design are limited. Further, the design tasks such virtual screening and molecular docking scoring/ranking the novel molecule accurately in drug design is technically challenging.

Some of the conventional techniques in drug design are employed and engineered by integrating in-silico, in-vitro, and in-vivo approaches. However, these conventional techniques are not accurate, nor efficient and are time consuming. Some of the conventional techniques in the drug design significantly mention employing Generative Artificial Intelligence (GenAI) and Artificial Intelligence (AI) models to design and identify novel molecules. However, most of these conventional techniques do not explicitly mention details related to workflow for identifying novel molecules by integrating GenAI with in-silico, in-vitro, and in-vivo approaches.

### SUMMARY

Embodiments of the present disclosure present technological improvements as solutions to one or more of the above-mentioned technical problems recognized by the inventors in conventional systems.

**In** an aspect, a processor-implemented method for dynamic drug design of a pharmacological target is provided. The method including the steps of: receiving (i) a pharmacological target for which a drug is to be designed, (ii) one or more protein and nucleic acid databases associated with the pharmacological target, (iii) one or more bibliographic databases associated with the pharmacological target, (iv) one or more publicly available small molecule databases associated with the pharmacological target, (v) one or more fragment libraries associated with a synthesis of molecule, (vi) one or more reaction rules associated with the synthesis of molecule, and (vii) one or more binding affinity databases associated with the pharmacological target; generating a plurality of target specific molecules associated with the pharmacological target, by employing one or more trained target specific molecule generation models based on one or more known drug properties associated with the pharmacological target, and a property threshold of each of the one or more known drug properties; identifying one or more lead molecules associated with the one or more known drug properties of the pharmacological target, from the plurality of target specific molecules; identifying one or more clustered same-core and unique-core molecules and one or more Absorption, Distribution, Metabolism, Excretion and Toxicity (ADMET) property filtered molecules, from the one or more lead molecules associated with the pharmacological target, using a clustering technique and one or more ADMET properties, respectively; determining one or more selective molecules that have similar drug-like mechanism from one or more common molecules using a pre-trained molecule mechanism determining model and a pre-trained multi-target machine learning (ML) model, wherein the one or more common molecules are obtained by identifying one or more molecules that are common in the one or more clustered same-core and unique-core molecules and the one or more ADMET property filtered molecules; determining one or more candidate molecules from the one or more selective molecules based on a molecule ranking of each of the one or more selective molecules; selecting one or more diverse potent molecules from the one or more candidate molecules based on a stability and one or more in-vitro experiments of each of the one or more candidate molecules; generating one or more lead molecules from the one or more diverse potent molecules using a scaffold hopping technique; iteratively perform a lead optimization cycle technique, on the one or more lead molecules, to obtain one or more optimized lead molecules; and selecting the drug-like molecule for the pharmacological target, using the one or more optimized lead molecules.

**In** another aspect, a system for dynamic drug design of a pharmacological target is provided. The system includes: a memory storing instructions; one or more Input/Output (I/O) interfaces; and one or more hardware processors coupled to the memory via the one or more I/O interfaces, wherein the one or more hardware processors are configured by the instructions to: receive (i) a pharmacological target for which a drug is to be designed, (ii) one or more protein and nucleic acid databases associated with the pharmacological target, (iii) one or more bibliographic databases associated with the pharmacological target, (iv) one or more publicly available small molecule databases associated with the pharmacological target, (v) one or more fragment libraries associated with a synthesis of molecule, (vi) one or more reaction rules associated with the synthesis of molecule, and (vii) one or more binding affinity databases associated with the pharmacological target; generate a plurality of target specific molecules associated with the pharmacological target, by employing one or more trained target specific molecule generation models based on one or more known drug properties associated with the pharmacological target, and a property threshold of each of the one or more known drug properties; identify one or more lead molecules associated with the one or more known drug properties of the pharmacological target, from the plurality of target specific molecules; identify one or more clustered same-core and unique-core molecules and one or more Absorption, Distribution, Metabolism, Excretion and Toxicity (ADMET) property filtered molecules, from the one or more lead molecules associated with the pharmacological target, using a clustering technique and one or more ADMET properties, respectively; determine one or more selective molecules that have similar drug-like mechanism from one or more common molecules using a pre-trained molecule mechanism determining model and a pre-trained multi-target machine learning (ML) model, wherein the one or more common molecules are obtained by identifying one or more molecules that are common in the one or more clustered same-core and unique-core molecules and the one or more ADMET property filtered molecules; determine one or more candidate molecules from the one or more selective molecules based on a molecule ranking of each of the one or more selective molecules; select one or more diverse potent molecules from the one or more candidate molecules based on a stability and one or more in-vitro experiments of each of the one or more candidate molecules; generate one or more lead molecules from the one or more diverse potent molecules using a scaffold hopping technique; iteratively perform a lead optimization cycle technique, on the one or more lead molecules, to obtain one or more optimized lead molecules; and select the drug-like molecule for the pharmacological target, using the one or more optimized lead molecules.

In yet another aspect, there are provided one or more non-transitory machine-readable information storage mediums comprising one or more instructions which when executed by one or more hardware processors cause:: receiving (i) a pharmacological target for which a drug is to be designed, (ii) one or more protein and nucleic acid databases associated with the pharmacological target, (iii) one or more bibliographic databases associated with the pharmacological target, (iv) one or more publicly available small molecule databases associated with the pharmacological target, (v) one or more fragment libraries associated with a synthesis of molecule, (vi) one or more reaction rules associated with the synthesis of molecule, and (vii) one or more binding affinity databases associated with the pharmacological target; generating a plurality of target specific molecules associated with the pharmacological target, by employing one or more trained target specific molecule generation models based on one or more known drug properties associated with the pharmacological target, and a property threshold of each of the one or more known drug properties; identifying one or more lead molecules associated with the one or more known drug properties of the pharmacological target, from the plurality of target specific molecules; identifying one or more clustered same-core and unique-core molecules and one or more Absorption, Distribution, Metabolism, Excretion and Toxicity (ADMET) property filtered molecules, from the one or more lead molecules associated with the pharmacological target, using a clustering technique and one or more ADMET properties, respectively; determining one or more selective molecules that have similar drug-like mechanism from one or more common molecules using a pre-trained molecule mechanism determining model and a pre-trained multi-target machine learning (ML) model, wherein the one or more common molecules are obtained by identifying one or more molecules that are common in the one or more clustered same-core and unique-core molecules and the one or more ADMET property filtered molecules; determining one or more candidate molecules from the one or more selective molecules based on a molecule ranking of each of the one or more selective molecules; selecting one or more diverse potent molecules from the one or more candidate molecules based on a stability and one or more in-vitro experiments of each of the one or more candidate molecules; generating one or more lead molecules from the one or more diverse potent molecules using a scaffold hopping technique; iteratively performing a lead optimization cycle technique, on the one or more lead molecules, to obtain one or more optimized lead molecules; and selecting the drug-like molecule for the pharmacological target, using the one or more optimized lead molecules.

In an embodiment, the one or more hardware processors (104) are configured to obtain the one or more target specific molecule generation models by training one or more Generative Artificial Intelligence (GenAI)-based models and one or more Artificial Intelligence (AI)-based models with the one or more protein and nucleic acid databases associated with the pharmacological target, the one or more bibliographic databases associated with the pharmacological target, the one or more publicly available small molecule databases associated with the pharmacological target, the one or more fragment libraries associated with the synthesis of molecule, the one or more reaction rules associated with the synthesis of molecule, and the one or more binding affinity databases associated with the pharmacological target, the one or more known drug properties associated with the pharmacological target, and the property threshold of each of the one or more known drug properties.

In an embodiment, the one or more known drug properties associated with the pharmacological target and the property threshold of each of the one or more known drug properties, are extracted from the one or more bibliographic databases, using a pre-trained known drug properties extraction model with a retrieval augmented generation (RAG) approach.

In an embodiment, the one or more lead molecules associated with the one or more known drug properties of the pharmacological target are identified from the plurality of target specific molecules, by: removing one or more duplicate molecules from the plurality of target specific molecules using one or more filtering techniques to obtain a first set of target specific molecules; removing one or more molecules having a toxic functional group, from the first set of target specific molecules using one or more rule-filtering techniques to obtain a second set of target specific non-toxic molecules; identifying one or more molecules that exhibit a high binding affinity for the pharmacological target from the second set of target specific non-toxic molecules using a molecular docking technique, to obtain a third set of target specific binding molecules; identifying one or more molecules that have active site residue interactions from the third set of target specific binding molecules using a target-ligand interactions library that eliminates molecules having unfavorable weak interactions, to obtain a fourth set of target specific binding molecules; and filtering the fourth set of target specific binding molecules using a multi-property optimization filter, to obtain the one or more lead molecules associated with the one or more known drug properties of the pharmacological target.

In an embodiment, the one or more clustered same-core and unique-core molecules from the one or more lead molecules associated with the pharmacological target, are identified using the clustering technique, by: identifying a first set of structurally same core molecules and a second set of structurally unique core molecules, from the one or more lead molecules associated with the pharmacological target, using a structural similarity technique; identifying a third set of property similar and structurally similar molecules from the first set of structurally same core molecules, using a dimensional reduction technique and a nearest neighbor approach; identifying a fourth set of property similar and structurally unique molecules from the second set of structurally unique core molecules, using the dimensional reduction technique and the nearest neighbor approach; identifying a fifth set of pharmacophore similar and structurally similar molecules from the third set of property similar and structurally similar molecules, using one or more molecular modelling techniques; and combining the fourth set of property similar and structurally unique molecules and the fifth set of pharmacophore similar and structurally similar molecules, to obtain the one or more clustered same-core and unique-core molecules.

In an embodiment, the one or more ADMET property filtered molecules are identified from the one or more lead molecules associated with the pharmacological target using the one or more ADMET properties, by: identifying one or more known drug molecules associated with the pharmacological target, from the one or more bibliographic databases and the one or more publicly available small molecule databases associated with the pharmacological target, using one or more known drug molecules identification models; extracting the one or more ADMET properties of each of the one or more known drug molecules associated with the pharmacological target, from the one or more chemical databases and the one or more bibliographic databases associated with the pharmacological target, using a pre-trained ADMET properties extraction model; predicting a property value of each of the one or more ADMET properties of each of the one or more lead molecules and each of the one or more known drug molecules associated with the pharmacological target, using a pre-trained ADMET property prediction model; and identifying the one or more ADMET property filtered molecules from the one or more lead molecules using a ADMET filter, based on the property value of each of the one or more ADMET properties of each of the one or more lead molecules using the property value of each of the one or more ADMET properties of each of the one or more known drug molecules associated with the pharmacological target.

In an embodiment, the molecule ranking of each of the one or more selective molecules is determined by: determining the selectivity ranking of each of the one or more selective molecules using the pre-trained multi-target machine learning (ML) model; and re-ranking the selectivity ranking of each of the one or more selective molecules using one of (i) quantum mechanical and molecular mechanical (QM and MM) technique and (ii) a molecular dynamics (MD) simulation and binding free energy calculations, to obtain the molecule ranking of each of the one or more selective molecules.

In an embodiment, selecting the one or more diverse potent molecules from the one or more candidate molecules based on the stability and the one or more in-vitro experiments of each of the one or more candidate molecules comprises by iteratively performing: identifying one or more stable molecules from the one or more candidate molecules based on the stability of each of the one or more candidate molecules through a Quantum mechanics-based drug stress testing; obtaining one or more first biologically evaluated potent lead molecules from the one or more stable molecules using the one or more in-vitro experiments, based on a biological activity; and selecting the one or more diverse potent molecules from the one or more first biologically evaluated potent lead molecules using one or more quantum mechanics based crystal structure prediction techniques.

In an embodiment, the one or more lead molecules are generated from the one or more diverse potent molecules using the scaffold hopping technique, by: generating one or more molecules having synthesizable fragments, from the one or more diverse potent molecules, using a scaffold hopping technique; generating one or more property optimized molecules, from the one or more diverse potent molecules, using a scaffold hopping technique-based molecule generation model; and selecting the one or more lead molecules from at least one of: (i) the one or more molecules having synthesizable fragments, and (ii) the one or more property optimized molecules, based on the affinity, a selectivity ranking, and the stability, wherein the affinity and the stability are determined using one or more of: (i) a Quantitative structure activity and property relationship (QSAR/QSPR) technique, a quantum mechanical and molecular mechanical (QM and MM) technique based docking, a free energy perturbation technique, and drug stress testing studies.

In an embodiment, the lead optimization cycle technique is iteratively performed on the one or more lead molecules to obtain the one or more optimized lead molecules, by: (a) determining a ADMET property result of each of the one or more lead molecules, using a pre-trained ADMET property result determining model; (b) determining an in-vitro and an in-vivo analysis result of each of the one or more lead molecules, using one or more in-vitro and in-vivo analysis techniques; and (c) iteratively performing the steps (a) and (b) until the one or more optimized lead molecules are obtained, based on the ADMET property result and the in-vitro and the in-vivo analysis result, using an active learning of the pre-trained ADMET property result determining model and a functional group modification of each of the one or more lead molecules based on an explainability of the pre-trained ADMET property result determining model.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 is an exemplary block diagram of a system for dynamic drug design of a pharmacological target, in accordance with some embodiments of the present disclosure.
FIGS. 2A and 2B illustrate exemplary flow diagrams of a processor-implemented method for dynamic drug design of a pharmacological target, using the system of FIG. 1, in accordance with some embodiments of the present disclosure.
FIG. 3 is a flowchart showing steps for identifying the one or more lead molecules associated with the one or more known drug properties of the pharmacological target, from the plurality of target specific molecules, using the system of FIG. 1, in accordance with some embodiments of the present disclosure.
FIG. 4 is a flowchart showing steps for identifying the one or more clustered same-core and unique-core molecules from the one or more lead molecules associated with the pharmacological target using the clustering technique, using the system of FIG. 1, in accordance with some embodiments of the present disclosure.
FIG. 5 is a flowchart showing steps for identifying the one or more ADMET property filtered molecules from the one or more lead molecules associated with the pharmacological target using the one or more ADMET properties, using the system of FIG. 1, in accordance with some embodiments of the present disclosure.
FIG. 6 is a flowchart showing steps for determining the molecule ranking of each of the one or more selective molecules, using the system of FIG. 1, in accordance with some embodiments of the present disclosure.
FIG. 7 is a flowchart showing steps for selecting the one or more diverse potent molecules from the one or more candidate molecules based on the stability and the one or more in-vitro experiments of each of the one or more candidate molecules, using the system of FIG. 1, in accordance with some embodiments of the present disclosure.
FIG. 8 is a flowchart showing steps for generating the one or more lead molecules from the one or more diverse potent molecules using the scaffold hopping technique, using the system of FIG. 1, in accordance with some embodiments of the present disclosure.
FIG. 9 is a flowchart showing steps for iteratively performing the lead optimization cycle technique on the one or more lead molecules to obtain the one or more optimized lead molecules, using the system of FIG. 1, in accordance with some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The present disclosure solves the technical problems in the art with the methods and systems for dynamic drug design of a pharmacological target. The methods and systems of the present disclosure make the drug design by integrating the in-silico, in-vitro and in-vivo approaches through the dynamic generative artificial intelligence (GenAI) and artificial intelligence (AI) technologies. The integration of in-silico (molecular modeling and AI), in-vitro and in-vivo approaches helps in designing the novel optimized lead molecules. Optimization and prediction of ADMET based on QM based descriptors helps in filtering the molecules.

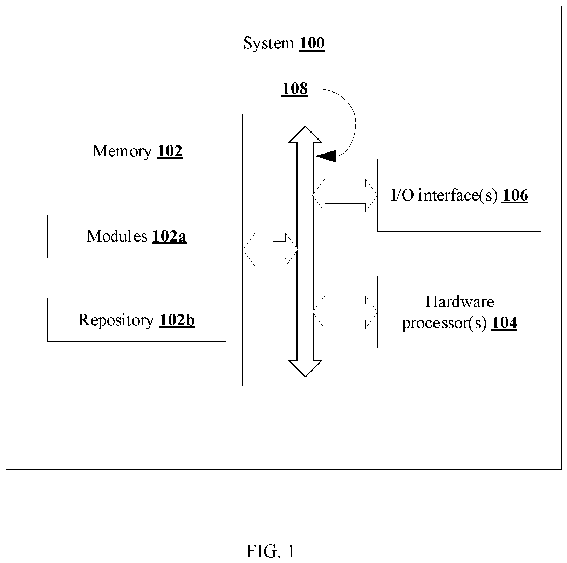

Referring now to the drawings, and more particularly to FIG. 1 through FIG. 9, where similar reference characters denote corresponding features consistently throughout the figures, there are shown preferred embodiments, and these embodiments are described in the context of the following exemplary systems and/or methods.

FIG. 1 is an exemplary block diagram of a system 100 for dynamic drug design of a pharmacological target, in accordance with some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface (GUI), and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computers, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. In an embodiment, the plurality of modules 102a can include various sub-modules (not shown in FIG. 1). Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external database may be periodically updated. For example, data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

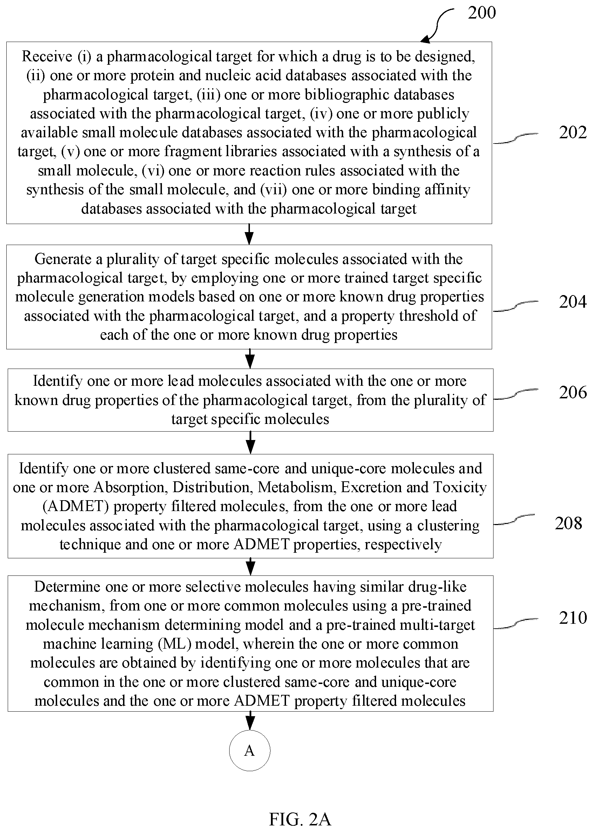

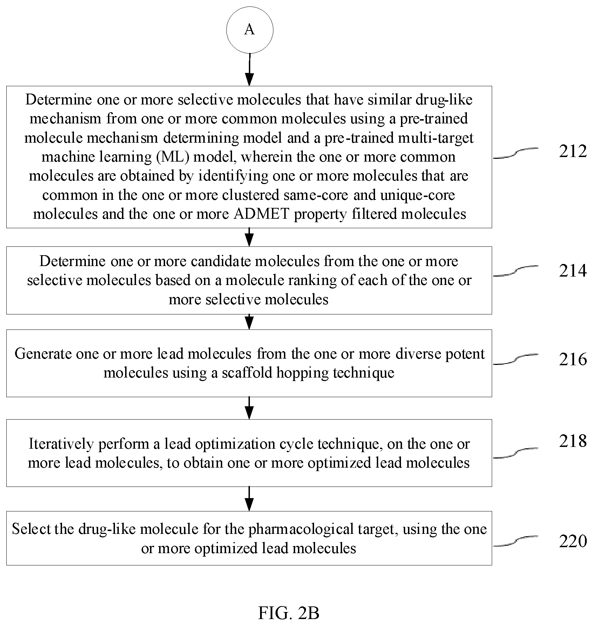

Referring to FIGS. 2A and 2B, components and functionalities of the system 100 are described in accordance with an example embodiment of the present disclosure. For example, FIGS. 2A and 2B illustrate exemplary flow diagrams of a processor-implemented method 200 for dynamic drug design of a pharmacological target, using the system 100 of FIG. 1, in accordance with some embodiments of the present disclosure. Although steps of the method 200 including process steps, method steps, techniques or the like may be described in a sequential order, such processes, methods, and techniques may be configured to work in alternate orders. **In** other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 202 of the method 200, the one or more hardware processors 104 of the system 100 are configured to receive a pharmacological target for which a drug is to be designed. **In** an embodiment, the pharmacological target is a biochemical entity to which the drug first binds in the body to elicit its effect. For example, the pharmacological target may be proteins such as receptors, enzymes, transporters, ion channels, or genetic material such as Deoxyribonucleic acid (DNA) and ribonucleic acid (RNA).

Along with the pharmacological target, the domain knowledge, research knowledge and other relevant information to the pharmacological target, such as one or more protein and nucleic acid databases, and one or more bibliographic databases are utilized to facilitate the generation of novel molecules meeting the specified pharmacological target.

Further, one or more publicly available small molecule databases, one or more fragment libraries associated with a synthesis of a small molecule, one or more reaction rules associated with the synthesis of the small molecule, and one or more binding affinity databases are also received, which are served as training data for generative models employed in generating novel molecules specifically designed to interact with the pharmacological target.

At step 204 of the method 200, the one or more hardware processors 104 of the system 100 are configured to generate a plurality of target specific molecules associated with the pharmacological target. In an embodiment, one or more trained target specific molecule generation models are employed to generate the plurality of target specific molecules. Further, the plurality of target specific molecules is generated based on one or more known drug properties associated with the pharmacological target, and a property threshold of each of the one or more known drug properties.

**In** an embodiment, the one or more target specific molecule generation models are obtained by training one or more Generative Artificial Intelligence (GenAI)-based models and one or more Artificial Intelligence (AI)-based models with the one or more protein and nucleic acid databases associated with the pharmacological target, the one or more bibliographic databases associated with the pharmacological target, the one or more publicly available small molecule databases associated with the pharmacological target, the one or more fragment libraries associated with the synthesis of molecule, the one or more reaction rules associated with the synthesis of molecule, and the one or more binding affinity databases associated with the pharmacological target, the one or more known drug properties associated with the pharmacological target, and the property threshold of each of the one or more known drug properties.

In an embodiment, the one or more Generative Artificial Intelligence (GenAI)-based models are Large Language Models (LLMs). By harnessing the power of LLMs, relevant information such as physicochemical properties or drug-like characteristics is extracted from research articles found within these databases. This automated process not only saves researchers valuable time but also enhances overall efficiency in the drug design and engineering process.

**In** an embodiment, the one or more known drug properties associated with the pharmacological target and the property threshold of each of the one or more known drug properties, are extracted from the one or more bibliographic databases, using a pre-trained known drug properties extraction model with a retrieval augmented generation (RAG) approach.

The RAG approach enables the integration of retrieved information with generative models, allowing for the production of target-specific novel molecules that possess desired properties. This combination of utilizing LLMs to extract pertinent data from bibliographic databases and integrating it with generative models enhances the workflow's ability to generate molecules with improved drug-like qualities and accelerates the drug discovery process.

At step 206 of the method 200, the one or more hardware processors 104 of the system 100 are configured to identify one or more lead molecules that exhibits the one or more known drug properties of the pharmacological target, from the plurality of target specific molecules obtained at step 204 of the method 200.

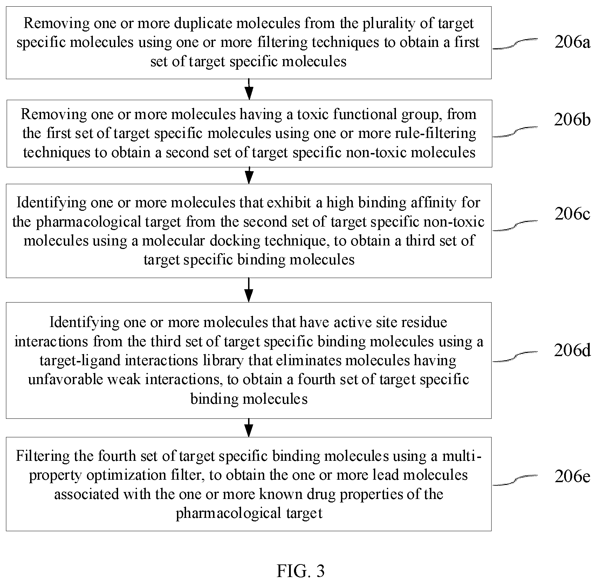

FIG. 3 is a flowchart showing steps for identifying the one or more lead molecules associated with the one or more known drug properties of the pharmacological target, from the plurality of target specific molecules, using the system 100 of FIG. 1, in accordance with some embodiments of the present disclosure. As shown in FIG. 3, identifying the one or more lead molecules associated with the one or more known drug properties of the pharmacological target from the plurality of target specific molecules are explained through steps 206a to 206e.

At step 206a, one or more duplicate molecules from the plurality of target specific molecules using one or more filtering techniques to obtain a first set of target specific molecules. The molecules that are duplicate or present for more than once in the plurality of target specific molecules are removed to make the first set of target specific molecules. In other words, the first set of target specific molecules comprises the molecules that are unique.

At step 206b, one or more molecules having a toxic functional group are removed from the first set of target specific molecules obtained at step 206a. The molecules that are toxic in nature (identified by the toxic functional group) are removed from the first set of target specific molecules. In an embodiment, one or more rule-filtering techniques are employed for removing the first set of target specific molecules that are having toxic functional group, to obtain a second set of target specific non-toxic molecules. Thus, the second set of target specific non-toxic molecules are the molecules that are non-toxic or toxic free. In an embodiment, rule-based filters are employed which apply predefined rules to exclude the compounds that do not conform to specific criteria. These rules could encompass various factors such as physicochemical properties, toxicity profiles, or structural features.

At step 206c, one or more molecules that exhibit a high binding affinity for the pharmacological target are identified from the second set of target specific non-toxic molecules obtained at step 206b. A molecular docking technique is applied for determining the binding affinity of each of the molecules present in the second set of target specific non-toxic molecules for the pharmacological target. The molecules that exhibit the high binding affinity out of the second set of target specific non-toxic molecules are formed as a third set of target specific binding molecules. In an embodiment, a computational screening is employed that evaluates the molecules' compatibility with the pharmacological target. Depending on the user's preference, this screening can be performed with or without considering interactions with water molecules.

At step 206d, one or more molecules that have active site residue interactions are identified from the third set of target specific binding molecules obtained at step 206c. In an embodiment, a target-ligand interactions library (a protein-ligand interactions library in case the target is a protein) is used to eliminate the molecules having unfavorable weak interactions, to obtain a fourth set of target specific binding molecules. Thus, the fourth set of target specific binding molecules are the molecules that exhibit the active site residue interactions. In this step, the molecules are filtered based on their predicted protein-ligand interactions, ensuring favorable binding interactions with the pharmacological target. This filter eliminates molecules with unfavorable or weak interactions, enhancing the likelihood of identifying potent ligands.

At step 206e, the one or more lead molecules associated with the one or more known drug properties of the pharmacological target are obtained by filtering the fourth set of target specific binding molecules, using a multi-property optimization filter. The multi-property optimization filter further refines the selection based on multiple desired properties, including but not limited to potency, selectivity, solubility, and bioavailability. Only molecules meeting the predefined optimization criteria are retained.

After the series of sequential filtrations through the steps 206a to 206e, the remaining molecules that have successfully passed are moved forward to the next steps of the workflow. These selected molecules represent the most promising candidates for further evaluation, optimization, and potential development as potential drug candidates.

At step 208 of the method 200, the one or more hardware processors 104 of the system 100 are configured to identify one or more clustered same-core and unique-core molecules and one or more Absorption, Distribution, Metabolism, Excretion and Toxicity (ADMET) property filtered molecules, from the one or more lead molecules identified at step 206 of the method 200.

In an embodiment, the one or more clustered same-core and unique-core molecules are the lead molecules that exhibit same-core and unique-core properties when they are clustered. In an embodiment, a clustering technique is employed to identify the one or more clustered same-core and unique-core molecules from the one or more lead molecules identified at step 206 of the method 200.

In an embodiment, the clustering technique such as a Butina clustering algorithm is used to classify molecules to identify two distinct clusters: one containing molecule with the same core structure as known drugs, and the other cluster consisting of molecules with unique core structures. The dimensionality reduction technique such as t-SNE is applied on Multi-Property Optimization (MPO) features to refine these clusters. The Near neighbor approaches are then used to select a specified number "N" of molecules from each cluster that closely resemble the known drugs in terms of MPO properties. Additionally, a structural similarity filter is applied to further refine the same core molecule selection, focusing on molecules with similar structures to the known drugs. The molecules with the same core structure as known drugs which got filtered based on structural similarity and "N" molecules from unique core cluster are grouped together as clustered molecules. The same core molecules are the molecules that contains the central moiety remain constant, while the unique core molecules are the molecules that contains a distinct central moiety.

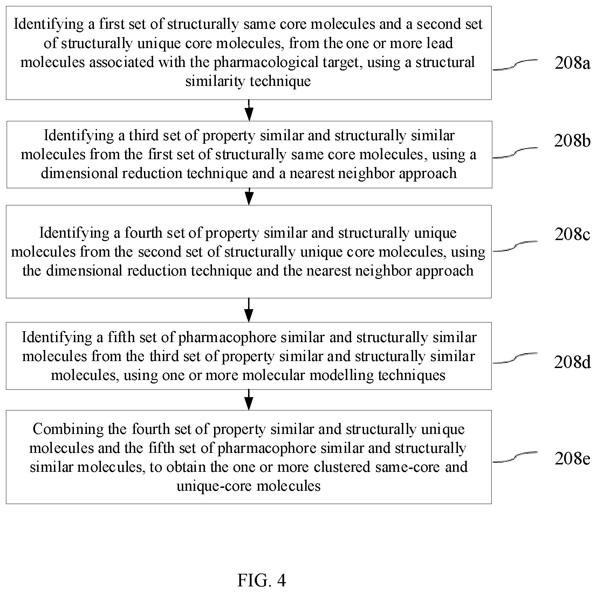

FIG. 4 is a flowchart showing steps for identifying the one or more clustered same-core and unique-core molecules from the one or more lead molecules associated with the pharmacological target, using the clustering technique, using the system 100 of FIG. 1, in accordance with some embodiments of the present disclosure. As shown in FIG. 4, identifying the one or more clustered same-core and unique-core molecules from the one or more lead molecules associated with the pharmacological target using the clustering technique is explained through steps 208a to 208e.

At step 208a, a first set of structurally same core molecules and a second set of structurally unique core molecules are identified from the one or more lead molecules obtained at step 206 of the method 200. A structural similarity technique is employed to compare the similarity of the molecules in terms of their structure and the core. The first set of structurally same core molecules are the molecules that are structurally same core molecules. The second set of structurally unique core molecules are the molecules that are structurally unique.

At step 208b, a third set of property similar and structurally similar molecules are identified from the first set of structurally same core molecules obtained at step 208a. the dimensional reduction technique and the nearest neighbor approach are employed to identify the molecules that are property similar and structurally similar out of the molecules in the first set of structurally same core molecules, to obtain the third set of property similar and structurally similar molecules.

At step 208c, a fourth set of property similar and structurally unique molecules are identified from the second set of structurally unique core molecules obtained at step 208a. The dimensional reduction technique and the nearest neighbor approach are employed to identify the molecules that are property similar and structurally unique out of the molecules in the second set of structurally unique core molecules, to obtain the fourth set of property similar and structurally unique molecules.

At step 208d, a fifth set of pharmacophore similar and structurally similar molecules are identified from the third set of property similar and structurally similar molecules obtained at step 208b. In an embodiment, one or more molecular modelling techniques are employed to identify the molecules that are pharmacophore similar and structurally similar out of the molecules in the third set of property similar and structurally similar molecules, to obtain the fifth set of pharmacophore similar and structurally similar molecules.

At step 208e, the fourth set of property similar and structurally unique molecules obtained at step 208c, and the fifth set of pharmacophore similar and structurally similar molecules obtained at step 208d are combined to obtain the one or more clustered same-core and unique-core molecules, using the clustering technique. Thus, the one or more clustered same-core and unique-core molecules are the molecules that are same-core molecules and the unique-core molecules.

In an embodiment, the one or more Absorption, Distribution, Metabolism, Excretion and Toxicity (ADMET) property filtered molecules are the lead molecules that satisfies the Absorption, Distribution, Metabolism, Excretion and Toxicity (ADMET) properties. Thus, one or more ADMET properties are used to filter the one or more lead molecules identified at step 206 of the method 200, to identify the ADMET property filtered molecules.

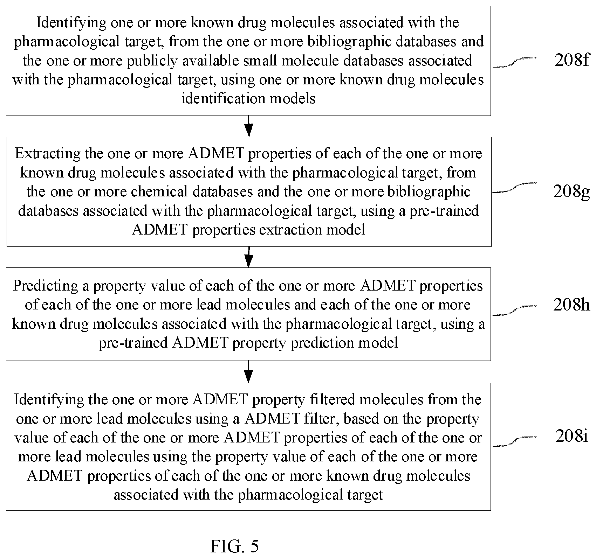

FIG. 5 is a flowchart showing steps for identifying the one or more ADMET property filtered molecules from the one or more lead molecules associated with the pharmacological target using the one or more ADMET properties, in accordance with some embodiments of the present disclosure. As shown in FIG. 5, identifying the one or more ADMET property filtered molecules from the one or more lead molecules associated with the pharmacological target using the one or more ADMET properties are explained through steps 208f to 208i.

At step 208f, one or more known drug molecules associated with the pharmacological target are identified from the one or more bibliographic databases and the one or more publicly available small molecule databases associated with the pharmacological target, using one or more known drug molecules identification models. The one or more known drug molecules are the drug molecules that are known in the art for the pharmacological target.

At step 208g, the one or more ADMET properties of each of the one or more known drug molecules associated with the pharmacological target are extracted. In an embodiment, a pre-trained ADMET properties extraction model is employed to extract the one or more ADMET properties of each of the one or more known drug molecules using the one or more chemical databases and the one or more bibliographic databases associated with the pharmacological target.

At step 208h, a property value of each of the one or more ADMET properties extracted at step 208g, of each of the one or more lead molecules and each of the one or more known drug molecules associated with the pharmacological target is predicted. In an embodiment, a pre-trained ADMET property prediction model is employed to predict the property value of each of the one or more ADMET properties.

At step 208i, the one or more ADMET property filtered molecules are identified from the one or more lead molecules identified at step 208f using a ADMET filter, based on the corresponding property value of each of the one or more ADMET properties of each of the one or more lead molecules and the one or more known drug molecules associated with the pharmacological target.

At step 210 of the method 200, the one or more hardware processors 104 of the system 100 are configured to determine one or more selective molecules that have similar drug-like mechanism from one or more common molecules. The one or more common molecules are obtained by identifying one or more molecules that are common in the one or more clustered same-core and unique-core molecules and the one or more ADMET property filtered molecules identified at step 208 of the method 200. More specifically, the one or more common molecules are the molecules that exhibits both the same-core and unique-core properties and ADMET properties.

The one or more selective molecules are obtained from the common molecules that have similar drug-like mechanism. In an embodiment, a pre-trained molecule mechanism determining model and a pre-trained multi-target machine learning (ML) model are used to determine the one or more selective molecules from one or more common molecules.

In an embodiment, the selectivity of the target-specific molecules is predicted by Gen AI algorithms using a screening approach involving Multi-target Classification and Regression-based machine learning models utilizing quantitative structure-property/activity relationships (QSAR/QSPR) techniques. To develop the multi-target machine learning models, molecules for all therapeutic targets are extracted from public chemical databases by leveraging the power of Large Language Models (LLMs) and the Retrieval-Augmented Generation (RAG) technique (15) to extract relevant data from databases such as Chembl, LINCS, Drugbank, CHEBI, among others. The extracted molecules undergo preprocessing to filter out those with binding affinity values. Measures such as dissociation constant (Kd), inhibitory constant (Ki), and half-maximal inhibitory concentration (IC50) are used to quantify the binding affinity.

Based on user-defined binding affinity thresholds, the molecules with these binding affinity values are classified into training and test sets. This classification process enables the development of multi-target machine learning models capable of predicting the interaction of molecules with each target. These models aid in identifying compounds with higher selectivity and in distinguishing between active and inactive compounds more effectively.

The implementation of these multi-target machine learning models enables rapid screening of several compounds, significantly reducing the time and cost involved in the initial stages of drug discovery. By predicting the selectivity of target-specific molecules, this approach enhances the understanding of their potential interactions and aids in identifying compounds with improved selectivity profiles. This analysis allows the user to prioritize molecules with similar mechanisms, ensuring better selectivity and minimizing the risk of adverse reactions. The outcome helps in identifying candidates that have a higher likelihood of success in subsequent stages of the drug design and engineering process.

At step 212 of the method 200, the one or more hardware processors 104 of the system 100 are configured to determine one or more candidate molecules from the one or more selective molecules obtained at step 210 of the method 200. The one or more candidate molecules are selected from the one or more selective molecules based on a molecule ranking of each of the one or more selective molecules.

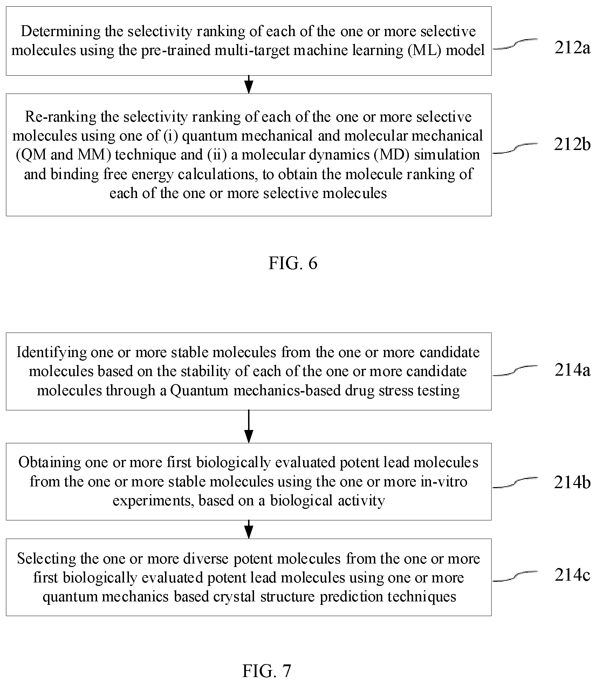

FIG. 6 is a flowchart showing steps for determining the molecule ranking of each of the one or more selective molecules, using the system 100 of FIG. 1, in accordance with some embodiments of the present disclosure. As shown in FIG. 6, determining the molecule ranking of each of the one or more selective molecules is explained through steps 212a to 212b.

At step 212a, the selectivity ranking of each of the one or more selective molecules is determined using the pre-trained multi-target machine learning (ML) model.

At step 212b, the selectivity ranking of each of the one or more selective molecules determined at step 212a are re-ranked using one of (i) quantum mechanical and molecular mechanical (QM and MM) technique and (ii) a molecular dynamics (MD) simulation and binding free energy studies, to obtain the molecule ranking of each of the one or more selective molecules. The selection of the Quantum mechanics and Molecular dynamics simulation techniques depends on the nature of the active site of the pharmacological target. This re-ranking method reduces the cost and time of lead selection process.

To obtain the QM optimized molecules, geometry optimization is performed using Quantum mechanics to obtain accurate molecular geometries, electronic structure, properties and energies to generate the QM optimized molecule or QM optimized conformer. The QM optimized conformer is used to perform molecular docking for finding the docked pose in the active site of the protein. The docked pose is re-optimized using DFT, MP2, CCSDT to obtain the QM derived properties like binding free energies, partial charges, dipole moments, singe point energies, HOMO-LUMO to re-rank the molecules. Several known molecules for all the therapeutic targets can be geometry optimized using the QM to develop machine learning models. These QM derived property-based machine learning models can help to accelerate the procedure of re-ranking molecules.

Alternatively, Molecular Dynamics Simulation Techniques and the free energy binding techniques are employed to simulate the dynamic behavior of the molecules over time, allowing for a deeper understanding of their structural stability, flexibility, and interactions in a realistic environment. This approach provides valuable information on the molecules' behavior and suitability for further development. Both of these evaluation methods aid in prioritizing the candidate molecules based on their calculated properties and behaviors. In an embodiment, scientific expertise and human evaluation are also used in the decision-making process. The human evaluation involves the careful assessment and ranking of the prioritized molecules by experts in the field. Their evaluation takes into account various factors such as scientific knowledge, experimental feasibility, potential therapeutic efficacy, and overall value. The human evaluation helps ensure a comprehensive assessment of the candidates and allows for further refinement and re-ranking of the prioritized molecules.

By combining scientific evaluation techniques, such as Quantum Mechanics Scoring or Molecular Dynamics Simulation, with human expertise, the workflow aims to identify the most promising molecules with the greatest potential for success in subsequent stages, leading to the development of effective and safe drug candidates.

At step 214 of the method 200, the one or more hardware processors 104 of the system 100 are configured to select one or more diverse potent molecules from the one or more candidate molecules obtained at step 212 of the method 200. The one or more diverse potent molecules are selected from the one or more candidate molecules based on a stability and one or more in-vitro experiments of each of the one or more candidate molecules.

FIG. 7 is a flowchart showing steps for selecting the one or more diverse potent molecules from the one or more candidate molecules based on the stability and the one or more in-vitro experiments of each of the one or more candidate molecules, using the system 100 of FIG. 1, in accordance with some embodiments of the present disclosure. As shown in FIG. 7, selecting the one or more diverse potent molecules from the one or more candidate molecules is explained through steps 214a to 214c.

At step 214a, one or more stable molecules from the one or more candidate molecules obtained step 212 of the method 200. The one or more stable molecules are the molecules that exhibit stability. A Quantum mechanics-based drug stress testing is performed to identify which molecules exhibit the stability out of the one or more candidate molecules to get the one or more stable molecules. This analysis helps understand the stability of the novel molecules, particularly under different environmental conditions and stress factors. If the molecules pass this testing, they proceed to the next step. If not, another molecule (22) is selected from the re-ranked set for further evaluation.

At step 214b, one or more first biologically evaluated potent lead molecules are identified from the one or more stable molecules obtained at step 214a, using the one or more in-vitro experiments, based on a biological activity. In this stage, protein and ligand binding experiments are performed using cell-based or biochemical assays. These experiments are designed to screen the computationally selected stable molecules and filter out lead molecules with high potency towards the specific target. If the molecules pass the in vitro biological screening process, they proceed to the next steps. However, if they do not pass, the models are retrained using experimental data, and new molecules are selected from the re-ranked set.

At step 214c, the one or more diverse potent molecules are selected from the one or more first biologically evaluated potent lead molecules obtained at step 214b, using one or more quantum mechanics based crystal structure prediction techniques.

The molecules that successfully pass the synthesis and in vitro evaluation are then subjected to Quantum Mechanics Crystal Structure Prediction. This technique predicts the crystal structures of the molecules based on experimental studies. From the in-vitro studies, 2-3 molecules are selected for lead optimization. The lead optimization phase focuses on refining the selected molecules to enhance their potency, selectivity, and overall drug-like properties. This step involves further modifications and iterations to optimize the candidate molecules, resulting in potential lead compounds for further development.

At step 216 of the method 200, the one or more hardware processors 104 of the system 100 are configured to generate one or more lead molecules from the one or more diverse potent molecules obtained at step 214 of the method 200. In an embodiment, a scaffold hopping technique is employed to generate the one or more lead molecules from the one or more diverse potent molecules.

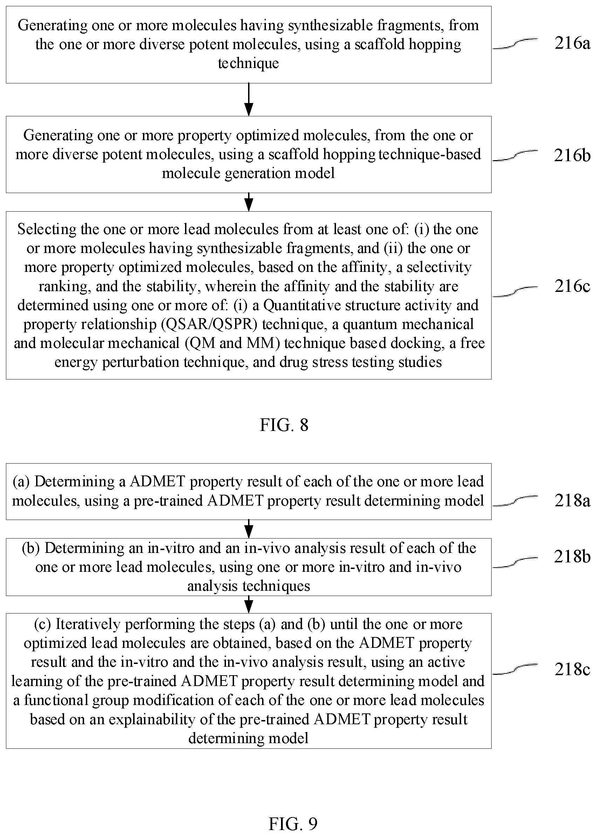

FIG. 8 is a flowchart showing steps for generating the one or more lead molecules from the one or more diverse potent molecules using the scaffold hopping technique, using the system 100 of FIG. 1, in accordance with some embodiments of the present disclosure. As shown in FIG. 8, generating the one or more lead molecules from the one or more diverse potent molecules is explained through steps 216a to 216c.

At step 216a, one or more molecules having synthesizable fragments are identified out of the one or more diverse potent molecules obtained at step 214 of the method 200. In an embodiment, a scaffold hopping technique is employed to identify the molecules that have synthesizable fragments.

The scaffold hopping technique is employed to generate novel analogs of the lead molecule that possess similar or desired pharmacological properties while potentially improving its potency, selectivity, and drug-like characteristics. Scaffold hopping is performed through screening fragment databases, such as ScafBank, eMolecules, Enamine, Mcule, and Chembridge, utilizing a fine-tuned Large Language Model (LLM) trained on the provided database. The LLM aids in suggesting alternative fragments for the given initial scaffold based on the properties of existing target-specific drugs, thus facilitating the identification of novel analogs. The generated analogs undergo ranking based on statistical analysis and screening approaches, focusing on the functional groups found in existing known target-specific molecules. By screening against known target-specific molecules from small molecule databases, it becomes easier to distinguish the generated molecules as either active or inactive.

At step 216b, the one or more property optimized molecules are generated from the one or more diverse potent molecules obtained at step 214 of the method 200. In an embodiment, a scaffold hopping technique-based molecule generation model is employed to generate the one or more property optimized molecules.

The GenAI-based scaffold hopping step involves the utilization of artificial intelligence techniques to enhance the process of exploring and optimizing drug scaffolds. During this step, a GenAI algorithm is employed to generate new and diverse scaffolds or fragments that can potentially serve as alternatives to the original scaffold of a lead molecule. The GenAI algorithm utilizes machine learning and computational methods to propose novel chemical structures that exhibit desirable properties, such as improved potency, selectivity, and drug-like characteristics. The algorithm is trained on large datasets of known chemical structures and their corresponding properties, enabling it to learn patterns and relationships that govern the structure-activity relationship (SAR) of compounds. By leveraging this knowledge, the algorithm can generate and evaluate a vast number of structurally diverse scaffolds or fragments. The GenAI-based scaffold hopping step also takes into consideration the specific requirements and goals of the drug optimization process. This enhances the likelihood of identifying analogs that possess the desired attributes and can potentially serve as improved drug candidates.

At step 216c, the one or more lead molecules are selected from at least one of: (i) the one or more molecules having synthesizable fragments generated at step 216a, and (ii) the one or more property optimized molecules generated at step 216b, based on the affinity, a selectivity ranking, and the stability of the corresponding molecule (the one or more molecules having synthesizable fragments and the one or more property optimized molecules). In an embodiment, the affinity and the stability of the molecules are determined using one or more of: (i) a Quantitative structure activity and property relationship (QSAR/QSPR) technique, a quantum mechanical and molecular mechanical (QM and MM) technique based docking, a free energy perturbation technique, and drug stress testing studies.

The selectivity ranking of each molecule is determined as explained at step 212a. The quantum mechanical and molecular mechanical (QM and MM) technique based docking and drug stress testing studies facilitates the exploration and analysis of the binding affinity, binding mode, and stability of the lead molecules with novel functional groups for a protein of interest. It is emphasized that the integration of Quantum Mechanics or Molecular Mechanics Docking into the workflow enables accurate predictions regarding the suitability of the lead molecules for further development by evaluating the binding affinity, binding mode, and the stability of the lead molecules.

In an embodiment, the molecules obtained from the Quantum Mechanics or Molecular Mechanics Docking is meticulously reviewed and evaluated by a user or team of experts with significant knowledge and expertise in the relevant field. This human evaluation involves scrutinizing various aspects such as the binding affinity, binding mode, and stability of the lead molecules with novel functional groups for the protein of interest.

During this filtering step, the experts consider numerous factors including the potential therapeutic efficacy, possible side effects, solubility, toxicity profiles, and other related parameters. Based on their comprehensive evaluation, the experts make informed decisions regarding the selection and prioritization of the most promising lead molecules for further development.

The incorporation of human evaluation in the workflow enables an additional layer of scrutiny and enhances the selection of lead molecules that hold the greatest potential for successful drug design and engineering. This filtering step, driven by human expertise, ensures the viability and potential of the lead molecules to progress to subsequent stages of the drug development process.

At step 218 of the method 200, the one or more hardware processors 104 of the system 100 are configured to obtain one or more optimized lead molecules from the one or more lead molecules obtained at step 216 of the method 200. In an embodiment, a lead optimization cycle technique is iteratively performed on the one or more lead molecules to obtain the one or more optimized lead molecules.

FIG. 9 is a flowchart showing steps for iteratively performing the lead optimization cycle technique on the one or more lead molecules to obtain the one or more optimized lead molecules, using the system 100 of FIG. 1, in accordance with some embodiments of the present disclosure. As shown in FIG. 9, iteratively performing the lead optimization cycle technique on the one or more lead molecules to obtain the one or more optimized lead molecules is explained through steps 218a to 218c.

Firstly, at step 218a, a ADMET property result of each of the one or more lead molecules is determined using a pre-trained ADMET property result determining model.

Then at step 218b, an in-vitro and an in-vivo analysis result of each of the one or more lead molecules is determined using one or more in-vitro and in-vivo analysis techniques.

Lastly at step 218c, the steps 218a and 218b are iteratively performed until one or more lead molecules, based on the ADMET property result and the in-vitro and the in-vivo analysis result, using an active learning of the pre-trained ADMET property result determining model and a functional group modification of each of the one or more lead molecules based on an explainability of the pre-trained ADMET property result determining model.

In an embodiment, the steps of Synthesis and Biological Evaluation of Molecule are performed simultaneously following the filtering based on Human Evaluation step. During this phase, all the lead molecule candidates identified during the previous step are subj ected to a series of laboratory assays, including but not limited to PAMPA, CACO2, CYPS, BBB, and others. These assays sequentially assess the characteristics and properties of the lead molecules, determining whether they exhibit negative or positive attributes.

Simultaneously, the lead molecules are also subjected to an AI model known as the Explainable ADMET Single or Multi-Property Prediction Model. ADMET properties of known drugs or inhibitors are obtained from databases such as FDA, Therapeutic Commons, and clinicaltrials.gov, utilizing Large Language Models (LLMs) and the Retrieval-Augmented Generation (RAG) technique to extract relevant information.

This AI model evaluates and predicts the ADMET (Absorption, Distribution, Metabolism, Excretion, and Toxicity) properties of the lead molecules, assisting in determining whether they possess positive or negative attributes in terms of ADMET properties.

Upon completion of these evaluations, the lead molecules falling into the category of being negative by both laboratory assays and the ADMET model are designated as True Negatives. Conversely, lead molecules identified as positive by both the laboratory assays and the ADMET model are designated as True Positives. Lead molecules that exhibit positive results in the laboratory assays but negative results in the ADMET model are referred to as False Negatives. Similarly, lead molecules display negative results in the laboratory assays but positive results in the ADMET model are referred to as False Positives.

The True Negative and False Positive lead molecules are then subjected to the step called "Functional Group Modification of Molecule Based on Lab Experiment and Explainability Models ". In this step, users can modify the functional groups of these molecules based on the insights gained from the laboratory experiments and the explainability models. Once the modifications are made, the molecules are re-fed into the process by re-entering the "Quantum Mechanics and Molecular Mechanics Docking " step.

On the other hand, the False Negative and False Positive lead molecules are fed into the step called "Active Learning Based on Lab Experiments and Explainability Models". In this step, users engage in a process known as active learning, where further refinement of the ADMET Model takes place. The goal is to enhance the model's accuracy and predictive capabilities through continuous refinement based on insights gained from laboratory experiments and the explainability models.

Finally, the molecules classified as False Negative and True Positive, which have successfully passed the biological evaluation, are identified as the optimized lead molecules. The workflow ensures a continuous feedback loop involving Al, human expertise, and laboratory experiments to obtain the optimized lead molecules with enhanced properties and efficacy. This comprehensive approach, integrating multiple elements, guarantees the generation of superior drug candidates. Thus, the present invention successfully combines AI, human evaluation, and laboratory experimentation to facilitate the development of the optimized lead molecules, setting it apart in the field of drug design and engineering.

At step 220 of the method 200, the one or more hardware processors 104 of the system 100 are configured to select the drug-like molecule for the pharmacological target, using the one or more optimized lead molecules obtained at step 218 of the method 200.

The methods and systems of the present disclosure design the drug for the given pharmacological target quickly, effectively, and efficiently by integrating the in-silico, in-vitro and in-vivo approaches through the dynamic GenAI and AI technology. The integration of in-silico (molecular modeling and AI), in-vitro and in-vivo approaches helps in designing the novel optimized lead molecules. Optimization and prediction of ADMET based on QM based descriptors helps in filtering the molecules. Further, Active learning of the models to refine the molecules, QM based scoring and ranking, crystal structure prediction and drug stress testing helps in identifying the candidate molecules that are most suitable in the effective drug design.

The methods and systems of the present disclosure integrates the artificial intelligence (AI), the Quantum and molecular mechanics into the lead design and optimization pipeline enhances the efficiency by improving data integration, automating processes, enhancing predictive models and creating dynamic feedback loops. This results in faster, more accurate and cost-effective identification and optimization of novel molecules ultimately accelerating the drug discovery process. Further, the methods and systems of the present disclosure provide the workflow for identifying the lead molecules and the drug design and the workflow of the present disclosure is a simple and a flexible process.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of the present disclosure herein address unresolved problems of dynamic drug design for the given pharmacological target by integrating the in-silico, in-vitro and in-vivo approaches through the dynamic GenAI and AI technologies. The integration of in-silico (molecular modeling and AI), in-vitro and in-vivo approaches helps in designing the novel optimized lead molecules. Optimization and prediction of ADMET based on QM based descriptors helps in filtering the molecules.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (200), comprising:
receiving, via one or more input/output (I/O) interfaces, (i) a pharmacological target for which a drug is to be designed, (ii) one or more protein and nucleic acid databases associated with the pharmacological target, (iii) one or more bibliographic databases associated with the pharmacological target, (iv) one or more publicly available small molecule databases associated with the pharmacological target, (v) one or more fragment libraries associated with a synthesis of a small molecule, (vi) one or more reaction rules associated with the synthesis of the small molecule, and (vii) one or more binding affinity databases associated with the pharmacological target (202);
generating, via the one or more hardware processors, a plurality of target specific molecules associated with the pharmacological target, by employing one or more trained target specific molecule generation models based on one or more known drug properties associated with the pharmacological target, and a property threshold of each of the one or more known drug properties (204);
identifying, via the one or more hardware processors, one or more lead molecules associated with the one or more known drug properties of the pharmacological target, from the plurality of target specific molecules (206);
identifying, via the one or more hardware processors, one or more clustered same-core and unique-core molecules and one or more Absorption, Distribution, Metabolism, Excretion and Toxicity (ADMET) property filtered molecules, from the one or more lead molecules associated with the pharmacological target, using a clustering technique and one or more ADMET properties, respectively (208);
determining, via the one or more hardware processors, one or more selective molecules having similar drug-like mechanism, from one or more common molecules using a pre-trained molecule mechanism determining model and a pre-trained multi-target machine learning (ML) model, wherein the one or more common molecules are obtained by identifying one or more molecules that are common in the one or more clustered same-core and unique-core molecules and the one or more ADMET property filtered molecules (210);
determining, via the one or more hardware processors, one or more candidate molecules from the one or more selective molecules based on a molecule ranking of each of the one or more selective molecules (212);
selecting, via the one or more hardware processors, one or more diverse potent molecules from the one or more candidate molecules based on a stability and one or more in-vitro experiments of each of the one or more candidate molecules (214);
generating, via the one or more hardware processors, one or more lead molecules from the one or more diverse potent molecules using a scaffold hopping technique (216);
iteratively performing, via the one or more hardware processors, a lead optimization cycle technique, on the one or more lead molecules, to obtain one or more optimized lead molecules (218); and
selecting, via the one or more hardware processors, the drug-like molecule for the pharmacological target, using the one or more optimized lead molecules (220).

2. The processor-implemented method as claimed in claim 1, wherein;
the one or more target specific molecule generation models are obtained by training one or more Generative Artificial Intelligence (GenAI)-based models and one or more Artificial Intelligence (AI)-based models with the one or more protein and nucleic acid databases associated with the pharmacological target, the one or more bibliographic databases associated with the pharmacological target, the one or more publicly available small molecule databases associated with the pharmacological target, the one or more fragment libraries associated with the synthesis of molecule, the one or more reaction rules associated with the synthesis of molecule, and the one or more binding affinity databases associated with the pharmacological target, the one or more known drug properties associated with the pharmacological target, and the property threshold of each of the one or more known drug properties, and
the one or more known drug properties associated with the pharmacological target and the property threshold of each of the one or more known drug properties, are extracted from the one or more bibliographic databases, using a pre-trained known drug properties extraction model with a retrieval augmented generation (RAG) approach.

3. The processor-implemented method as claimed in claim 1, wherein;
identifying the one or more lead molecules associated with the one or more known drug properties of the pharmacological target, from the plurality of target specific molecules, comprises:
removing one or more duplicate molecules from the plurality of target specific molecules using one or more filtering techniques to obtain a first set of target specific molecules (206a);
removing one or more molecules having a toxic functional group, from the first set of target specific molecules using one or more rule-filtering techniques to obtain a second set of target specific non-toxic molecules (206b);
identifying one or more molecules that exhibit a high binding affinity for the pharmacological target from the second set of target specific non-toxic molecules using a molecular docking technique, to obtain a third set of target specific binding molecules (206c);
identifying one or more molecules that have active site residue interactions from the third set of target specific binding molecules using a target-ligand interactions library that eliminates molecules having unfavorable weak interactions, to obtain a fourth set of target specific binding molecules (206d); and
filtering the fourth set of target specific binding molecules using a multi-property optimization filter, to obtain the one or more lead molecules associated with the one or more known drug properties of the pharmacological target (206e), and
the one or more clustered same-core and unique-core molecules are identified from the one or more lead molecules associated with the pharmacological target, using the clustering technique, by:
identifying a first set of structurally same core molecules and a second set of structurally unique core molecules, from the one or more lead molecules associated with the pharmacological target, using a structural similarity technique (208a);
identifying a third set of property similar and structurally similar molecules from the first set of structurally same core molecules, using a dimensional reduction technique and a nearest neighbor approach (208b);
identifying a fourth set of property similar and structurally unique molecules from the second set of structurally unique core molecules, using the dimensional reduction technique and the nearest neighbor approach (208c);
identifying a fifth set of pharmacophore similar and structurally similar molecules from the third set of property similar and structurally similar molecules, using one or more molecular modelling techniques (208d); and
combining the fourth set of property similar and structurally unique molecules and the fifth set of pharmacophore similar and structurally similar molecules, to obtain the one or more clustered same-core and unique-core molecules (208e).

4. The processor-implemented method as claimed in claim 1, wherein the one or more ADMET property filtered molecules are identified from the one or more lead molecules associated with the pharmacological target using the one or more ADMET properties, by:
identifying one or more known drug molecules associated with the pharmacological target, from the one or more bibliographic databases and the one or more publicly available small molecule databases associated with the pharmacological target, using one or more known drug molecules identification models (208f);
extracting the one or more ADMET properties of each of the one or more known drug molecules associated with the pharmacological target, from the one or more chemical databases and the one or more bibliographic databases associated with the pharmacological target, using a pre-trained ADMET properties extraction model (208g);
predicting a property value of each of the one or more ADMET properties of each of the one or more lead molecules and each of the one or more known drug molecules associated with the pharmacological target, using a pre-trained ADMET property prediction model (208h); and
identifying the one or more ADMET property filtered molecules from the one or more lead molecules using a ADMET filter, based on the property value of each of the one or more ADMET properties of each of the one or more lead molecules using the property value of each of the one or more ADMET properties of each of the one or more known drug molecules associated with the pharmacological target (208i).

5. The processor-implemented method as claimed in claim 1, wherein the molecule ranking of each of the one or more selective molecules is determined by:
determining the selectivity ranking of each of the one or more selective molecules using the pre-trained multi-target machine learning (ML) model (212a); and
re-ranking the selectivity ranking of each of the one or more selective molecules using one of (i) quantum mechanical and molecular mechanical (QM and MM) technique and (ii) a molecular dynamics (MD) simulation and binding free energy calculations, to obtain the molecule ranking of each of the one or more selective molecules (212b).

6. The processor-implemented method as claimed in claim 1, wherein;
selecting the one or more diverse potent molecules from the one or more candidate molecules based on the stability and the one or more in-vitro experiments of each of the one or more candidate molecules comprises by iteratively performing:
identifying one or more stable molecules from the one or more candidate molecules based on the stability of each of the one or more candidate molecules through a Quantum mechanics-based drug stress testing (214a);
obtaining one or more first biologically evaluated potent lead molecules from the one or more stable molecules using the one or more in-vitro experiments, based on a biological activity (214b); and
selecting the one or more diverse potent molecules from the one or more first biologically evaluated potent lead molecules using one or more quantum mechanics based crystal structure prediction techniques (214c), and
generating the one or more lead molecules from the one or more diverse potent molecules using the scaffold hopping technique, further comprising:
generating one or more molecules having synthesizable fragments, from the one or more diverse potent molecules, using a scaffold hopping technique (216a);
generating one or more property optimized molecules, from the one or more diverse potent molecules, using a scaffold hopping technique-based molecule generation model (216b); and
selecting the one or more lead molecules from at least one of: (i) the one or more molecules having synthesizable fragments, and (ii) the one or more property optimized molecules, based on the affinity, a selectivity ranking, and the stability, wherein the affinity and the stability are determined using one or more of: (i) a Quantitative structure activity and property relationship (QSAR/QSPR) technique, a quantum mechanical and molecular mechanical (QM and MM) technique based docking, a free energy perturbation technique, and drug stress testing studies (216c).

7. The processor-implemented method as claimed in claim 1, wherein iteratively performing the lead optimization cycle technique on the one or more lead molecules to obtain the one or more optimized lead molecules, further comprising:
(a) determining a ADMET property result of each of the one or more lead molecules, using a pre-trained ADMET property result determining model (218a);
(b) determining an in-vitro and an in-vivo analysis result of each of the one or more lead molecules, using one or more in-vitro and in-vivo analysis techniques (218b); and
(c) iteratively performing the steps (a) and (b) until the one or more optimized lead molecules are obtained, based on the ADMET property result and the in-vitro and the in-vivo analysis result, using an active learning of the pre-trained ADMET property result determining model and a functional group modification of each of the one or more lead molecules based on an explainability of the pre-trained ADMET property result determining model (218c).

8. A system (100), comprising:
a memory (102) storing instructions;
one or more input/output (I/O) interfaces (106);
one or more hardware processors (104) coupled to the memory (102) via the one or more I/O interfaces (106), wherein the one or more hardware processors (104) are configured by the instructions to:
receive, via the one or more I/O interfaces (106), (i) a pharmacological target for which a drug is to be designed, (ii) one or more protein and nucleic acid databases associated with the pharmacological target, (iii) one or more bibliographic databases associated with the pharmacological target, (iv) one or more publicly available small molecule databases associated with the pharmacological target, (v) one or more fragment libraries associated with a synthesis of a small molecule, (vi) one or more reaction rules associated with the synthesis of the small molecule, and (vii) one or more binding affinity databases associated with the pharmacological target;
generate a plurality of target specific molecules associated with the pharmacological target, by employing one or more trained target specific molecule generation models based on one or more known drug properties associated with the pharmacological target, and a property threshold of each of the one or more known drug properties;
identify one or more lead molecules associated with the one or more known drug properties of the pharmacological target, from the plurality of target specific molecules;
identify one or more clustered same-core and unique-core molecules and one or more Absorption, Distribution, Metabolism, Excretion and Toxicity (ADMET) property filtered molecules, from the one or more lead molecules associated with the pharmacological target, using a clustering technique and one or more ADMET properties, respectively;
determine one or more selective molecules having similar drug-like mechanism from one or more common molecules using a pre-trained molecule mechanism determining model and a pre-trained multi-target machine learning (ML) model, wherein the one or more common molecules are obtained by identifying one or more molecules that are common in the one or more clustered same-core and unique-core molecules and the one or more ADMET property filtered molecules;
determine one or more candidate molecules from the one or more selective molecules based on a molecule ranking of each of the one or more selective molecules;
select one or more diverse potent molecules from the one or more candidate molecules based on a stability and one or more in-vitro experiments of each of the one or more candidate molecules;
generate one or more lead molecules from the one or more diverse potent molecules using a scaffold hopping technique;
iteratively perform a lead optimization cycle technique, on the one or more lead molecules, to obtain one or more optimized lead molecules; and
select the drug-like molecule for the pharmacological target, using the one or more optimized lead molecules.

9. The system as claimed in claim 8, wherein the one or more hardware processors (104) are configured to;
obtain the one or more target specific molecule generation models by training one or more Generative Artificial Intelligence (GenAI)-based models and one or more Artificial Intelligence (AI)-based models with the one or more protein and nucleic acid databases associated with the pharmacological target, the one or more bibliographic databases associated with the pharmacological target, the one or more publicly available small molecule databases associated with the pharmacological target, the one or more fragment libraries associated with the synthesis of molecule, the one or more reaction rules associated with the synthesis of molecule, and the one or more binding affinity databases associated with the pharmacological target, the one or more known drug properties associated with the pharmacological target, and the property threshold of each of the one or more known drug properties, and
extract the one or more known drug properties associated with the pharmacological target and the property threshold of each of the one or more known drug properties, from the one or more bibliographic databases, using a pre-trained known drug properties extraction model with a retrieval augmented generation (RAG) approach.

10. The system as claimed in claim 8, wherein the one or more hardware processors (104) are configured to;
identify the one or more lead molecules associated with the one or more known drug properties of the pharmacological target, from the plurality of target specific molecules, by:
removing one or more duplicate molecules from the plurality of target specific molecules using one or more filtering techniques to obtain a first set of target specific molecules;
removing one or more molecules having a toxic functional group, from the first set of target specific molecules using one or more rule-filtering techniques to obtain a second set of target specific non-toxic molecules;
identifying one or more molecules that exhibit a high binding affinity for the pharmacological target from the second set of target specific non-toxic molecules using a molecular docking technique, to obtain a third set of target specific binding molecules;
identifying one or more molecules that have active site residue interactions from the third set of target specific binding molecules using a target-ligand interactions library that eliminates molecules having unfavorable weak interactions, to obtain a fourth set of target specific binding molecules; and
filtering the fourth set of target specific binding molecules using a multi-property optimization filter, to obtain the one or more lead molecules associated with the one or more known drug properties of the pharmacological target, and
identify the one or more clustered same-core and unique-core molecules from the one or more lead molecules associated with the pharmacological target, using the clustering technique, by:
identifying a first set of structurally same core molecules and a second set of structurally unique core molecules, from the one or more lead molecules associated with the pharmacological target, using a structural similarity technique;
identifying a third set of property similar and structurally similar molecules from the first set of structurally same core molecules, using a dimensional reduction technique and a nearest neighbor approach;
identifying a fourth set of property similar and structurally unique molecules from the second set of structurally unique core molecules, using the dimensional reduction technique and the nearest neighbor approach;
identifying a fifth set of pharmacophore similar and structurally similar molecules from the third set of property similar and structurally similar molecules, using one or more molecular modelling techniques; and
combining the fourth set of property similar and structurally unique molecules and the fifth set of pharmacophore similar and structurally similar molecules, to obtain the one or more clustered same-core and unique-core molecules.

11. The system as claimed in claim 8, wherein the one or more hardware processors (104) are configured to identify the one or more ADMET property filtered molecules from the one or more lead molecules associated with the pharmacological target using the one or more ADMET properties, by:
identifying one or more known drug molecules associated with the pharmacological target, from the one or more bibliographic databases and the one or more publicly available small molecule databases associated with the pharmacological target, using one or more known drug molecules identification models;
extracting the one or more ADMET properties of each of the one or more known drug molecules associated with the pharmacological target, from the one or more chemical databases and the one or more bibliographic databases associated with the pharmacological target, using a pre-trained ADMET properties extraction model;
predicting a property value of each of the one or more ADMET properties of each of the one or more lead molecules and each of the one or more known drug molecules associated with the pharmacological target, using a pre-trained ADMET property prediction model; and
identifying the one or more ADMET property filtered molecules from the one or more lead molecules using a ADMET filter, based on the property value of each of the one or more ADMET properties of each of the one or more lead molecules using the property value of each of the one or more ADMET properties of each of the one or more known drug molecules associated with the pharmacological target.

12. The system as claimed in claim 8, wherein the one or more hardware processors (104) are configured to determine the molecule ranking of each of the one or more selective molecules, by:
determining the selectivity ranking of each of the one or more selective molecules using the pre-trained multi-target machine learning (ML) model; and
re-ranking the selectivity ranking of each of the one or more selective molecules using one of (i) quantum mechanical and molecular mechanical (QM and MM) technique and (ii) a molecular dynamics (MD) simulation and binding free energy calculations, to obtain the molecule ranking of each of the one or more selective molecules.

13. The system as claimed in claim 8, wherein the one or more hardware processors (104) are configured to;
select the one or more diverse potent molecules from the one or more candidate molecules based on the stability and the one or more in-vitro experiments of each of the one or more candidate molecules comprises by iteratively performing:
identifying one or more stable molecules from the one or more candidate molecules based on the stability of each of the one or more candidate molecules through a Quantum mechanics-based drug stress testing;
obtaining one or more first biologically evaluated potent lead molecules from the one or more stable molecules using the one or more in-vitro experiments, based on a biological activity; and
selecting the one or more diverse potent molecules from the one or more first biologically evaluated potent lead molecules using one or more quantum mechanics based crystal structure prediction techniques, and
generate the one or more lead molecules from the one or more diverse potent molecules using the scaffold hopping technique, by:
generating one or more molecules having synthesizable fragments, from the one or more diverse potent molecules, using a scaffold hopping technique;
generating one or more property optimized molecules, from the one or more diverse potent molecules, using a scaffold hopping technique-based molecule generation model; and
selecting the one or more lead molecules from at least one of: (i) the one or more molecules having synthesizable fragments, and (ii) the one or more property optimized molecules, based on the affinity, a selectivity ranking and the stability, wherein the affinity and the stability are determined using one or more of: (i) a Quantitative structure activity and property relationship (QSAR/QSPR) technique, a quantum mechanical and molecular mechanical (QM and MM) technique based docking, a free energy perturbation technique, and drug stress testing studies.

14. The system as claimed in claim 8, wherein the one or more hardware processors (104) are configured to iteratively perform the lead optimization cycle technique on the one or more lead molecules to obtain the one or more optimized lead molecules, by:
(a) determining a ADMET property result of each of the one or more lead molecules, using a pre-trained ADMET property result determining model;
(b) determining an in-vitro and an in-vivo analysis result of each of the one or more lead molecules, using one or more in-vitro and in-vivo analysis techniques; and
(c) iteratively performing the steps (a) and (b) until the one or more optimized lead molecules are obtained, based on the ADMET property result and the in-vitro and the in-vivo analysis result, using an active learning of the pre-trained ADMET property result determining model and a functional group modification of each of the one or more lead molecules based on an explainability of the pre-trained ADMET property result determining model.

15. One or more non-transitory machine-readable information storage mediums comprising one or more instructions which when executed by one or more hardware processors cause:
receiving (i) a pharmacological target for which a drug is to be designed, (ii) one or more protein and nucleic acid databases associated with the pharmacological target, (iii) one or more bibliographic databases associated with the pharmacological target, (iv) one or more publicly available small molecule databases associated with the pharmacological target, (v) one or more fragment libraries associated with a synthesis of a small molecule, (vi) one or more reaction rules associated with the synthesis of the small molecule, and (vii) one or more binding affinity databases associated with the pharmacological target;
generating a plurality of target specific molecules associated with the pharmacological target, by employing one or more trained target specific molecule generation models based on one or more known drug properties associated with the pharmacological target, and a property threshold of each of the one or more known drug properties;
identifying one or more lead molecules associated with the one or more known drug properties of the pharmacological target, from the plurality of target specific molecules;
identifying one or more clustered same-core and unique-core molecules and one or more Absorption, Distribution, Metabolism, Excretion and Toxicity (ADMET) property filtered molecules, from the one or more lead molecules associated with the pharmacological target, using a clustering technique and one or more ADMET properties, respectively;
determining one or more selective molecules having similar drug-like mechanism, from one or more common molecules using a pre-trained molecule mechanism determining model and a pre-trained multi-target machine learning (ML) model, wherein the one or more common molecules are obtained by identifying one or more molecules that are common in the one or more clustered same-core and unique-core molecules and the one or more ADMET property filtered molecules;
determining one or more candidate molecules from the one or more selective molecules based on a molecule ranking of each of the one or more selective molecules;
selecting one or more diverse potent molecules from the one or more candidate molecules based on a stability and one or more in-vitro experiments of each of the one or more candidate molecules;
generating one or more lead molecules from the one or more diverse potent molecules using a scaffold hopping technique;
iteratively performing a lead optimization cycle technique, on the one or more lead molecules, to obtain one or more optimized lead molecules; and
selecting the drug-like molecule for the pharmacological target, using the one or more optimized lead molecules.
